# EUROPEAN PATENT APPLICATION

(11) **EP 3 378 503 A2**
(43) Date of publication of application: **26.09.2018**
(21) Application number: 18275044.8
(22) Date of filing: 23.03.2018
(51) Int. Cl.: A61L 27/02, A61L 27/54, A61L 27/58, A61L 24/00, A61L 24/02

(54) **CALCIUM BASED CLINICAL MATERIAL WITH ANTIMICROBIAL PROPERTIES AND METHOD OF FORMING FOR PREVENTION OR TREATMENT OF INFECTION**

(30) Priority: 24.03.2017 GB 201704688
(71) Applicant: Biocomposites Limited, Staffordshire ST5 5NL (GB)
(72) Inventor: Laycock, Phillip, Sandbach, Cheshire CW11 4SW (GB); Cooper, John, Crewe, Cheshire CW3 9BJ (GB); Waters, Russell, Audlem, Cheshire CW3 0BA (GB); Colclough, John, Stoke on Trent, Staffordshire ST3 7WH (GB)
(74) Representative: Wilson Gunn

(57) **Abstract**

Disclosed is an absorbable, calcium based, clinical material with antimicrobial properties and a method of forming for the prevention or treatment of infection within hard and soft tissue within or on a patient's body.

## Description

### FIELD OF THE INVENTION

The present invention concerns a clinical material that inhibits the growth of a broad range of bacterial, fungal and protozoa pathogens with little or no potential to produce a build-up of resistance in those pathogens. The clinical material can be placed at the site of infection to treat the infection or assist in the prevention of infection without the need of antibiotics. The clinical material is completely absorbable by the body of a patient.

### BACKGROUND OF INVENTION

Infection is a serious complication of many clinical conditions and surgical procedures. The site of infection or potential infection can be localized in the patient's hard tissue or soft tissue, or it can be adjacent to a surgically implanted prosthesis such as a knee or hip implant, or an implanted device such as a pacemaker or breast implant, or a wound such as a diabetic foot ulcer or pressure ulcers.

The overuse, and particularly misuse, of antibiotics is increasingly leading to the development of antibiotic resistant pathogens. This means that the concentrations of antibiotics in the body which were previously sufficient to treat infections are no longer as effective. The increasingly high serum concentrations required to be effective can result in unacceptable systemic toxicity effects. Multidrug resistant and extensively drug resistant pathogens are becoming increasingly difficult, if not impossible, to treat through a systemic delivery route.

Medical implants can be prone to bacterial adhesion either from contamination during surgery or from haematogenous seeding post-implantation. These bacteria can then form a biofilm which is particularly resistant to both the host defences and antimicrobial therapy. The minimum inhibitory concentration (MIC) for bacterial biofilms can be at least 100 times the concentration required for the antibiotic to kill the same bacteria in their planktonic or free-floating form.

The application of antimicrobials directly at the site of an infection offers many advantages including providing high local concentrations which may exceed the MIC and even the minimum biofilm eradication concentration (MBEC) for many pathogens while at the same time ensuring that serum levels remain low thus minimising the potential for systemic toxicity effects.

Many devices that are surgically implanted in to a patient's body or topically applied to the surface of a wound can support microbial attachment, growth and biofilm formation. Microbial contamination of surgically implanted or topically applied devices can occur either at the time of surgery or through haematogenous seeding post-operatively. Following microbial attachment and colonisation, subsequent biofilm formation can lead to chronic infection with high rates of morbidity and mortality.

In addition, the costs associated with treating surgical site infections (SSIs) and implant associated infections (IAIs) impose a significant financial and logistical burden upon the healthcare providers. This problem is being exacerbated by the increasing incidence of antimicrobial resistant pathogens and the lack of new antimicrobial agents.

In the United States, it was estimated that there were 722,000 healthcare-associated infections (HAIs) in acute care hospitals in 2011. The number of deaths caused by HAIs during hospitalisation was estimated at 75,000¹. In addition, the costs associated with SSIs and IAIs impose a significant financial and logistical burden upon the healthcare providers. In one report from the United States it was found that the annual cost of treating HAIs ranges from 24.4 billion to 33.8 billion US dollars². This problem is being exacerbated by the increasing incidence of antimicrobial resistant pathogens and the lack of new antimicrobial agents.

Chronic wound infections (CWIs) as seen in chronic skin and soft tissue wounds including, but not limited to, diabetic foot ulcers, pressure ulcers and venous stasis ulcers are estimated to affect 5.7 million patients in the United States and the treatment of these CWIs can cost up to 20 billion dollars annual3. The current manner for treating CWIs is debridement of the wound and treating the infection with antibiotics.

Diabetic foot ulcers have an incidence of two percent per year in diabetic patients. The cost of care for patients suffering from diabetic foot ulcers in the United States cost an average of $52,000 US dollars for Medicare services total reimbursement per year⁴. Treatment for this type of ulcer again involves the debridement of the area and systemic antibiotics. The estimated lower limb amputation rate for people suffering from a diabetic foot ulcer is estimated at 50% to 70%.⁵

The current mainstay of treatment for infection is the use of antibiotics. However, the emergence of bacterial resistance means the efficacy of antibiotics is at risk and the reducing pipeline of new antibiotics is a worrying trend for today's clinicians. This emergence of resistant bacteria has been caused by the overuse and the misuse of antibiotics⁶. Therefore, there is an increasingly urgent need for more effective ways to treat infections within patients.

Materials having antimicrobial properties that are absorbable by the patient's body can help to clear an established infection at the site of their implantation, can be used prophylactically at a surgical or wound site, or can be applied topically at an external wound site on its own or as a wound dressing. A material which fully resorbs in a clinically meaningful timeframe can negate the need for an additional surgical procedure for its removal. A non-absorbable material, such as poly-methyl methacrylate (PMMA), which is often used to deliver antibiotics to protect against infection, generally requires a second operation for its removal. It is potentially possible that this second operation can cause further complications for the patient.

In the treatment of infected hard or soft tissue, it is important that all the devitalized and necrotic tissue is removed surgically. However, this removal of tissue will create what is known as 'dead space'. If this dead space is not managed effectively it can fill with tissue fluid or a blood clot, resulting in a seroma or hematoma. This will disrupt and separate the tissue or facial planes, thus resulting in arrested healing. The warm and moist environment of a seroma or hematoma can effectively act as the perfect culture environment for microbial growth. It is therefore important to obliterate the dead space.

It has been established that calcium sulfate, in paste, slurry, putty, gel, pellet, granule, bead or set paste form, is safe to use within a dead space environment or for dead space management. In a soft tissue site, the calcium sulfate resorbs safely into the body over a period of several days to several weeks to be replaced by new vital tissue. In addition, the presence of a calcium sulfate based clinical material with antimicrobial properties can prevent, reduce or eliminate the potential for microbial colonization.

A substance that is reported to be resorbable but is not suitable as an antimicrobial material is Bioglass. Bioglass is an implantable material composed of silicon dioxide, sodium oxide, calcium oxide and phosphorous pentoxide in different formulations. Commercially available Bioglass products for implantation will bind to bone and soft tissue.

Research has investigated the ability of different formulations of bioactive glass (BAG)-S53P4 to interfere with bacterial biofilm produced on prosthetic material by Methicillin Resistant *Staphylococcus aureus* (MRSA) and multi-drug-resistant *Pseudomonas aeruginosa.* The results demonstrated a reduction in biomass and total cell volume.⁷

In-vivo, bioglass converts to a hydroxyapatite like material and bonds to bone and soft tissue. The slower absorption profile makes this type of material unsuitable for active sites of infection and soft tissue sites, especially adjacent to cobalt chrome implant surfaces where it presents a scratching risk.

The problem to be solved is how to provide a clinical material for implantation or topical application, which may be used in hard or in soft tissue sites, is safe for use in a dead space environment or for dead space management, is fully absorbable in a clinically meaningful time period, which displays antimicrobial properties for the prevention or treatment of infection and exhibits little or no adverse tissue response or systemic complications.

### DETAILED DESCRIPTION OF THE INVENTION

Disclosed is an absorbable, clinical material with antimicrobial properties comprising a resorbable carrier material, the carrier material being selected from acidified calcium sulfate, a calcium phosphate, collagen, cancellous and cortical allograft bone, cancellous and cortical autograft bone, a resorbable polymer, or a composite material comprising any two or more thereof; and a method of forming for the prevention or treatment of infection within hard and soft tissue within or on a patient's body.
A clinically applicable and biocompatible, fully absorbable clinical material having antimicrobial properties is provided; where the clinical material inhibits microbial (bacterial, fungal or protozoa) colonisation, growth and subsequent biofilm formation in a simple and effective manner thus preventing or treating infection in hard and/or soft tissue sites.

The clinical material of the present invention may be used prophylactically or as a form of treatment in infected sites in a range of medical conditions including, but not limited to: surgical site infections, infected diabetic foot ulcers, prosthetic joint infections, open fractures, mediastinitis, vascular graft infection sites, infected pacemaker pockets or infections in penile implant or breast implant prostheses.

According to one embodiment of the invention, the clinical material of the invention typically further comprises a carboxylic salt of an alkali or alkaline earth metal. The clinical material may also further comprise a carboxylic acid.

Sodium or potassium salts of a number of carboxylic acids having antimicrobial properties including acetic acid, sorbic acid, propionic acid, valeric acid, butyric acid, benzoic acid, malic acid, ascorbic acid, glycolic acid, lactic acid, formic acid, caproic acid, enanthic acid, caprylic acid, pelargonic acid, capric acid, undecylic acid and lauric acid, have been used to control the growth of bacteria and extend the shelf life of meat, fish and poultry⁸⁻¹³. In addition, they have been demonstrated to possess antibacterial properties against *Staphylococcus aureus* and *Yersinia enterocolitica*¹⁴, *Listeria monocytogenes***¹⁵**, *Escherichia coli¹⁶* and *Clostridium botulinum*¹⁷*.*

Many of these salts are widely available and listed by the United States Food and Drug Administration (FDA) as Generally Recognized As Safe (GRAS) as food additives. These materials may also be identified as inactive ingredients in approved drug products.

Sodium acetate is the sodium salt of acetic acid. It is an inexpensive chemical that has a wide range of uses, including as a food additive or a laboratory reagent. Sodium acetate is added to food to help prevent bacterial growth.

Sodium acetate has been shown to be effective in inhibiting microbial growth. In addition, the combination of at least, but typically, 1.74% sodium lactate and typically 1.74% sodium acetate was demonstrated to reduce bacterial growth¹⁸.

Carboxylic acids are very important compounds biologically with many being involved in metabolic processes. Many carboxylic acids are also present in the foods and drinks we ingest. Acetate is a biologically acceptable chemical moiety which is a natural product of metabolism. It is involved in several important metabolic processes. At physiological pH values, acetic acid is usually fully ionised to acetate. The acetyl group, derived from acetic acid, is fundamental to all forms of life. When bound to coenzyme A, it is central to the metabolism of carbohydrates and fats. Similarly, lactic acid plays a role in several biochemical processes. Lactate is one of the main components of the intravenous fluids lactated Ringer's solution and Hartmann's solution together with sodium and potassium cations.

Sorbic acid and its salts are antimicrobial agents often used as preservatives in food and drinks to prevent the growth of mold, yeast, and fungi. Sorbic acid has a pH of 4.76, similar to acetic acid.

Acidic calcium sulfate (ACS) is a material known in the food industry. It is used to treat meat, poultry and fish products. It is a very acidic (pH 1.0-1.5) blend of calcium hydroxide, sulphuric acid and calcium sulfate and is used to decrease pathogen levels, as well as to extend the shelf life of the treated products. ACS with propionic and lactic acid and lactates has been used as a post-processing dipping solution to control *Listeria monocytogenes* on frankfurters¹⁹. However, the highly acidic nature and relatively low purity of these food grade materials makes them totally unsuitable for application on or within the human body. Highly acidic materials can attack bone and destroy soft tissue structures. Surprisingly, despite the considerable length of time that acetic acid and lactic acid have been used in the food industry, there is little data about the development of microbial resistance to these compounds. This lack of data may be viewed as a good indication that resistance development is probably not a major problem.

It has been demonstrated that an acidic environment can assist in wound healing by controlling the wound infection, increasing antimicrobial activity, altering protease activity, releasing oxygen, reducing toxicity of bacterial end products, and enhancing epithelization and angiogenesis.²⁰

The calcium phosphate carrier material may comprise, by way of non-limiting examples, a calcium phosphate selected from beta tricalcium phosphate, hydroxyapatite, monocalcium phosphate, dicalcium phosphate (dibasic calcium phosphate), tricalcium phosphate (tribasic calcium phosphate or tricalcic phosphate, sometimes referred to as calcium phosphate or calcium orthophosphate, whitlockite), octacalcium phosphate, dicalcium diphosphate, calcium triphosphate, tetracalcium phosphate. The calcium phosphate carrier material may be acidified or not acidified, as desired.

The resorbable polymer may comprise, by way of non-limiting examples, hydroxypropyl methylcellulose, carboxymethyl cellulose, polylactic acid (PLA), poly-L-lactide (PLLA), poly-D-lactide (PDLA), polycaprolactone, or chitosan.

The composite carrier material may comprise any two or more materials selected from the list of carrier materials defined hereinabove.

According to one embodiment, the resorbable carrier material comprises acidified calcium sulfate and/or a calcium phosphate, such as tricalcium phosphate or hydroxyapatite.

Prolonged exposure of bacteria or fungi to sub-inhibitory concentrations of antibiotics or antimycotics drives the development of resistance build-up in those pathogens.

A clinical material which does not contain antibiotic or antifungal drugs but is, however, effective in inhibiting the colonisation and/or growth of a broad spectrum of pathogens, including bacteria, fungi and protozoa while having little or no systemic adverse effects and little potential for inducing bacterial resistance build-up can offer significant benefit in certain infected, or potentially infected, sites on or within a patient's body.

The antimicrobial properties of the present invention provide an absorbable clinical material that can prevent or treat infection in a patient with or without the supplemental use of antibiotics. Thus, the clinical material can be used in or at infected sites that are poorly vascularised and where systemic antibiotics may not be effective.

The clinical material of the present invention typically comprises calcium sulfate, an aqueous liquid, together with a carboxylic salt of an alkali of alkaline earth metal and an optional carboxylic acid. The aqueous liquid may comprise a dilute solution of acetic or lactic acids, typically about 2% or 3% or 4% concentration mixed with water.

The calcium compound of the clinical material may comprise one or more of the following types of calcium sulfate: calcium sulfate hemihydrate, calcium sulfate dihydrate, anhydrous calcium sulfate, alone or in combination with calcium phosphate or calcium carbonate.

Where the carboxylic salt of the present invention may be selected from an alkali or alkaline earth metal of a low molecular mass, typically below 200 daltons, more typically between about 40 to about 200 daltons, wherein the carboxylic acid and is selected from sodium, potassium, magnesium or calcium cations alone or in combination, combined with acetate, ascorbate, benzoate, butyrate, citrate, formate, fumarate, glycolate, lactate, malate, maleate, oxalate, propionate, salicylate, sorbate, tartrate or valerate anions, or a combination of any two or more thereof.

The carboxylic acid of the present invention may be selected from the group including acetic acid, sorbic acid, propionic acid, valeric acid, butyric acid, benzoic acid, malic acid, ascorbic acid, glycolic acid, lactic acid, formic acid, caproic acid, enanthic acid, caprylic acid, pelargonic acid, capric acid, undecylic acid and lauric acid, or a combination of any two or more thereof.

The clinical material will have an acidic pH in the range of 3 to 6.5; more preferably an acidic pH in the range of 4 to 5.

According to one embodiment of the invention, the clinical material comprises a high purity calcium sulfate combined with sodium diacetate together with acetic acid and having a pH of between 4 and 6 which can be fully and safely absorbed by the patient's body.

According to another embodiment of the invention the clinical material comprises a high purity calcium sulfate combined with lactic acid solution together with L-lactic acid sodium salt and having a pH between 4 and 6 which can be fully and safely absorbed by the patient's body.

Alternatively, the clinical material may be provided as separate powder and liquid components which can be mixed at the time of surgery or treatment of a patient. The powder may comprise calcium sulfate hemihydrate together with an alkali or alkaline earth salt of a low molecular mass carboxylic acid while the liquid component comprises an aqueous solution of a carboxylic acid.

The material may be implanted as a paste to hydrate and set *in situ* or it may be moulded in to suitable shapes, such as beads, which may then be implanted when set. Alternatively, it may be incorporated in to a wound dressing, as a paste, to be applied topically, thus enabling versatility in treatment.

The clinical material comprises a salt of an alkali or alkaline earth carboxylic acid. The carboxylic acid has a low molecular mass, preferably between 40 to 200 daltons, more preferably between 50 to 100 daltons. Higher molecular mass carboxylic acids become increasingly less soluble, more hydrophobic and having little or no antimicrobial activity, which renders them unsuitable for the present invention.

Another medical grade calcium sulfate (Osteoset®, Wright Medical), for example, contains 1.5% calcium stearate. This is a high molecular mass, waxy solid, hydrophobic and insoluble in water. It is used as a pressing aid. Stearic acid has a molecular mass of 284 daltons which is higher than required by the clinical material of the present invention.

The calcium compound of the clinical material of the present invention is preferably calcium sulfate hemihydrate, which may be alpha-hemihydrate or beta-hemihydrate. The calcium sulfate hemihydrate is more preferably alpha-hemihydrate.

Preferably the calcium sulfate has a purity of not less than 98% and conforms to the requirements of ASTM F2224.

The clinical material of the present invention may contain an accelerant to speed-up the rate of hydration and thus hardening of the clinical material. The accelerant may consist of calcium sulfate dihydrate, potassium sulfate, sodium sulfate or sodium chloride present as either a powder in the powder components or as a solution in the aqueous mixing solution.

The present invention provides a clinical material which is safe to be implanted or used topically in the patient. The clinical material of the present invention may be used in hard tissue or soft tissue sites within or on the patient's body for the prevention or treatment of infection or may be used topically as an antimicrobial wound dressing. The present invention reduces the potential for microbial colonisation and subsequent biofilm formation and thus helps protect wound-healing and prevent the development of chronic infection.

The material of the invention may also contain one or more antibacterial agents, or one or more antifungal medicines, or one or more antineoplastic and immunosuppressives, or one or more analgesics. Any of these components may be added alone or in combination with each other.

Examples of antibacterial agents include, but are not limited to, any of the following, either alone or in combination: Amikacin, Amoxicillin, Amoxicillin/clavulanic acid (amoxicillin + clavulanic acid), Ampicillin, Azithromycin, Aztreonam, Benzathine benzylpenicillin, Benzylpenicillin, Cefalexin, Cefazolin, Cefepime, Cefixime, Cefotaxime, Ceftaroline fosamil, Ceftolazane/tazobactam, Ceftazidime, Ceftriaxone, Cefuroxime, Chloramphenicol, Ciprofloxacin, Clarithromycin, Clindamycin, Cloxacillin, Colistimethate sodium, Daptomycin, Doripenem, Doxycycline, Ertapenem, Erythromycin, Flucloxacillin, Flucytosine, Fusidic Acid, Gentamicin, Imipenem/cilastatin, Levofloxacin, Linezolid, Meropenem, Metronidazole, Moxifloxacin, Nafcillin, Nitrofurantoin, Penicillin, Phenoxymethylpenicillin (penicillin V), Piperacillin/tazobactam, Procaine benzylpenicillin, Rifampicin, Spectinomycin, Streptomycin, Tedizolid, Teicoplainin, Temocillin, Tigecycline, Tobramycin, Trimethoprim/sulfamethoxazole, Trimethoprim, Vancomycin.

Examples of antifungal medicines include, but are not limited to, any of the following, either alone or in combination: Amphotericin B, Anidulafungin, Caspofungin, Clotrimazole, Fluconazole, Flucytosine, Griseofulvin, Isavuconazole, Itraconazole, Micafungin, Nystatin, Posaconazole, Voriconazole.

Examples of antineoplastic and immunosuppressives include cytotoxic and adjuvant medicines, which include but are not limited to, any of the following, either alone or in combination: All-trans retinoic acid (tretinoin), Allopurinol, Asparaginase, Bendamustine, Bleomycin, Calcium folinate, Capecitabine, Carboplatin, Chlorambucil, Cisplatin, Cyclophosphamide, Cytarabine, Dacarbazine, Dactinomycin, Dasatinib, Daunorubicin, Docetaxel, Doxorubicin, Etoposide, Filgrastim, Fludarabine, Fluorouracil, Gemcitabine, Hydroxycarbamide, Ifosfamide, Imatinib, Irinotecan, Mercaptopurine, Mesna, Methotrexate, Oxaliplatin, Paclitaxel, Procarbazine, Rituximab, Thioguanine, Trastuzumab, Vinblastine, Vincristine, Vinorelbine, Zoledronic acid.

Examples of analgesics include, but are not limited to, any of the following, either alone or in combination: Bupivacaine, Lidocaine, Lidocaine/epinephrine.

The method and materials are simple, safe and inexpensive, thus negating the need for the use of expensive and potentially toxic antimicrobial compounds such as antibiotics.

The table below shows the Zone of Inhibition (ZOI) for the clinical material compared to two antibiotics for the treatment or prevention of *Pseudomonas aeruginosa.* Clear zone diameters measured in millimetres.

| | ZOI against *Pseudomonas aeruginosa* | | **ZOI Achieved with clinical material as disclosed** |
|---|---|---|---|
| **ABX** | **Susceptible** | **Resistant** | |
| Gentamicin*¹ | ≥15 | <15 | |
| Tobramycin*¹ | ≥16 | <16 | |
| Gentamicin*² | ≥15 | ≤12 | |
| Tobramycin*² | ≥15 | ≤12 | |
| Calcium sulfate combined with sodium diacetate and acetic acid (pH 4.68) (Example 3) | | | 15 - 16 |

The table below shows the ZOI for the clinical material of the present invention compared to four antibiotics for the treatment or prevention of *Staphylococcus aureus.*

| | ZOI against *Staphylococcus aureus* | | **ZOI Achieved with clinical material as disclosed** |
|---|---|---|---|
| **ABX** | **Susceptible** | **Resistant** | |
| Amikacin*¹ | ≥18 | <16 | |
| Tigecycline*¹ | ≥18 | <18 | |
| Gentamicin*¹ | ≥18 | <18 | |
| Tobramycin*¹ | ≥18 | <18 | |
| Calcium sulfate combined with lactic acid solution and L-lactic | | | 9 - 19 |
| acid sodium salt (pH 4.82) (example 4) | | | |

| | | | |
|---|---|---|---|
| *¹Based on the European committee on antimicrobial susceptibility testing clinical breakpoints for bacteria v7.0 (Jan 2017).²¹ *²Based on the American Society for Microbiology Kirby-Bauer disk diffusion susceptibility test protocol.²² | | | |

The data demonstrates that the clinical material of the present invention shows a ZOI equivalent or greater than that given by the noted antibiotic. This is particularly advantageous as it demonstrates that it is possible to achieve the same level of antimicrobial activity using the material of the present invention as is observed when using an antibiotic, but without the need to use an antibiotic. This resolves the issue of a resistance to antibiotics building up if the antibiotic is not being used.

The clinical material of the present invention may be used where there is infection of a medical device and may be injected into the surgical site without the need for further surgery.

The clinical material of the present invention may be placed in the surgical site at the time of the operation as a preventative measure - prophylactic use. It may be applied topically to wound sites to assist in the healing process.

The clinical material of the present invention may be used to treat infections caused by a wide range of pathogens including, but not limited to, *Propionibacterium acnes, Staphylococcus epidermidis, MRSA, Pseudomonas aeruginosa, Acinetobacter baumannii and Candida albicans.*

The clinical material of the present invention may be in the form of a paste, slurry, putty, gel, powder, cream, aerosol, solution, or set beads, granules or pellets or any other form or shape suitable for incorporation into the treatment site that would be recognised by a person skilled in this technical field. The clinical material of the invention may also be applied to, or soaked into, a bandage or wound dressing; this is particularly envisaged when the clinical material is in the form of a solution.

According to further embodiments the clinical material of the present invention can be used to prevent bacterial colonisation and infection at either soft or hard tissue sites within or on a patient's body.

According to a further embodiment of the present invention, there is provided a kit of parts for the production of a clinical material having antimicrobial properties for the treatment and prevention of infection within or on a patient's body; this kit of parts comprising a resorbable carrier material having antimicrobial properties, the carrier material comprising one of acidified calcium sulfate, a calcium phosphate, collagen, cancellous and cortical allograft bone, cancellous and cortical autograft bone, a resorbable polymer, or a composite material comprising any two or more thereof; an alkali or alkaline earth metal salt of a low molecular mass carboxylic acid; a hydrating solution comprising a low molecular mass carboxylic acid, and the tools to mix and apply the material.

According to an embodiment of the invention, a kit of parts is provided where the carrier material, such as calcium sulfate, is about 40 wt% to about 100 wt% of the clinical material more preferably where the carrier material is about 90 wt% to about 98 wt% of the clinical material. Where the carrier material is calcium sulfate, , it may be a calcium sulfate hemihydrate comprising between about 1 wt% to about 10 wt% carboxylic acid salt, more preferably about 2 wt% to about 5 wt% of carboxylic acid salt and further comprising about 1 wt% to about 10wt% carboxylic acid solution having a low molecular mass of between about 40 to about 200 daltons, more preferably about 2 wt% to about 5 wt%, carboxylic acid having a low molecular weight of between about 40 to about 200 daltons.

The clinical material of the present invention is absorbable within or on the patient's body over a period of time, typically within about 2 to about 12 weeks, more typically between about 4 to about 12 weeks when implanted in hard tissue within a patient's body. In soft tissue sites within or on a patient's body, the clinical material resorption occurs more rapidly, typically within about 2 to about 4 weeks.

### DEFINITIONS

Absorbable - can be broken down by biological activity and be absorbed within the patient Acidic pH - a pH value of less than 7.

ASTM F2224 - Standard Specification for High Purity Calcium Sulfate Hemihydrate or Dihydrate for Surgical Implants.

Anaerobic - describes an environment that either has no or almost no oxygen.

Antibiotic - describes a compound or substance that kills or slows down the growth of bacteria.

Antimicrobial - substance that kills or inhibits the growth of microorganisms such as bacteria, fungi, or protozoa.

Beads - Cylindrical, spherical or hemispherical form in sizes ranging from 3 to 9mm diameter.

Bacteria - a large group of unicellular microorganisms which can cause disease.

Biofilm - A Community of micro-organisms, attached to a surface and embedded within a self-produced extra-cellular matrix, which are extremely hard to detect and eradicate.

Blood Agar - Culture media for *Propionibacterium* species containing general nutrients and 5% sheep blood.

Carboxylic acid - an organic acid that contains one or more carboxyl groups.

Clinical - refers to the treatment of a patient.

Clinical breakpoints or breakpoints - antimicrobial concentrations used to interpret results of susceptibility testing to define isolates as susceptible, intermediate or resistant to a particular antimicrobial substance.

Dalton - unit of molecular mass equivalent to grams per mole (g/mol).

Dead space - is defined as the residual tissue void after tissue excision or loss.

Debridement or debrided - describes a surgical technique where dead, necrotic and infected tissue is surgically excised thus creating a void or 'dead space'.

Fungus - - a single-celled or multicellular organism; that feeds by absorbing organic molecules from its surroundings.

Hard tissue - bony tissue in a human or animal subject including bone and teeth.

Impurity - a constituent, not deliberately added, which impairs the purity of something, a contaminant.

Infection - is a condition within a subject's body, where there is the presence of one or more pathogens.

Low molecular mass organic acid or low molecular mass - describes an organic acid having a molecular mass typically not exceeding approximately 200 daltons.

Microbe - a bacterium causing disease or fermentation.

Microorganism - a bacterium or fungus.

Patient - a human or animal subject who is receiving or is to receive medical treatment.

Purity level or high purity - refers to the subject matter having no impurities contained within it therefore it only contains the subject matter, i.e. calcium sulfate.

Resorbable - refers to a material which resorbs in the human or animal body.

Sabouraud Dextrose agar - growth media for culturing fungi.

Soft tissue - connective tissue, other than hard tissue, that connects, supports, or surrounds other structures and organs of the body. It includes fibrous tissue, muscle and adipose tissue.

Treatment - the medical care given by a doctor or surgeon to a patient for an illness or injury. Tryptone soya agar or TSA refers to a growth media for the culturing of bacteria.

TSA plates - Triptone Soya Agar - growth media for the culturing of bacteria placed into a petri dish.

Zone of Inhibition (ZOI) refers to a clear region around a paper disc saturated with an antimicrobial agent on the agar surface where the clear region is an indication of either the absence or the effective inhibition of microbial growth by the antimicrobial agent.

Comprising or any cognate word specifies the presence of stated features, steps, or integers or components, but does not preclude the presence or addition of one or more other features, steps, integers, components or groups thereof. The expressions; "consists of' or "consists essentially of" or cognates may be embraced within "comprises" or cognates, wherein "consists essentially of' permits inclusion of substances not materially affecting the characteristics of the composition to which it applies.

### BRIEF DESCRIPTION OF THE DRAWINGS

The included figures show the microbiological response of organisms to antimicrobial calcium based materials formed into 6mm diameter beads against 8 microbial species in triplicate; *Staphylococcus aureus* ATCC 6538, *Staphylococcus epidermidis* ATCC 1228, MRSA NCTC 12493, *Pseudomonas aeruginosa* NCTC 13437, *Acinetobacter baumannii* NCTC 13424, *Enterococcus faecium* NCTC 12202, *Propionibacterium acnes* NCTC 737, *Candida albicans* ATCC 10231.

Agar plates were seeded with 0.2ml of a suspension of the relevant organism containing approximately 10⁸ cfu/ml. The plates were transferred to an incubator operating at 33 ±2 °C for 30 minutes. The plates were then removed from the incubator and the beads placed on the surface. The plates were then incubated again at 33 ±2 °C, after which they were removed from the incubator and examined for the absence of growth as seen by a clear zone around the test sample.

### DETAILED DESCRIPTION OF THE DRAWINGS

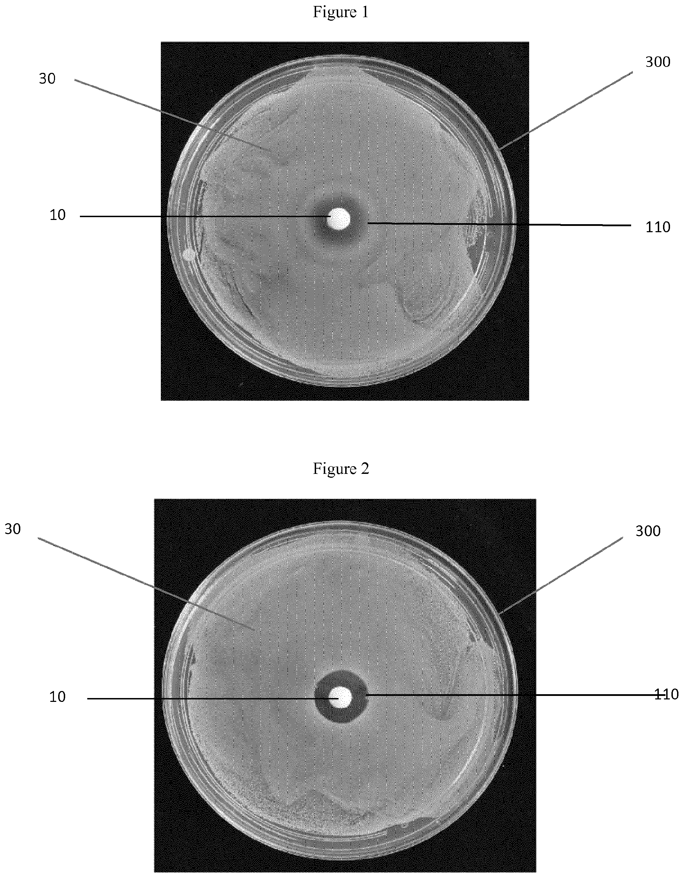

Figure 1 shows the antimicrobial properties of a bead 10 of the clinical material of the present invention showing a ZOI 110 of *Staphylococcus epidermidis* 30 with calcium sulfate combined with sodium diacetate and acetic acid (pH 4.83) incubated on Tryptone soya agar (TSA) plate 300 at 33 ±2 °C for 24 hours (hrs).
Figure 2 shows the antimicrobial properties of a bead 10 of the clinical material of the present invention showing a ZOI 110 of *Staphylococcus epidermidis* 30 with calcium sulfate combined with lactic acid solution and L-lactic acid sodium salt (pH 4.82) incubated on TSA plate 300 at 33 ±2 °C for 24 hrs.

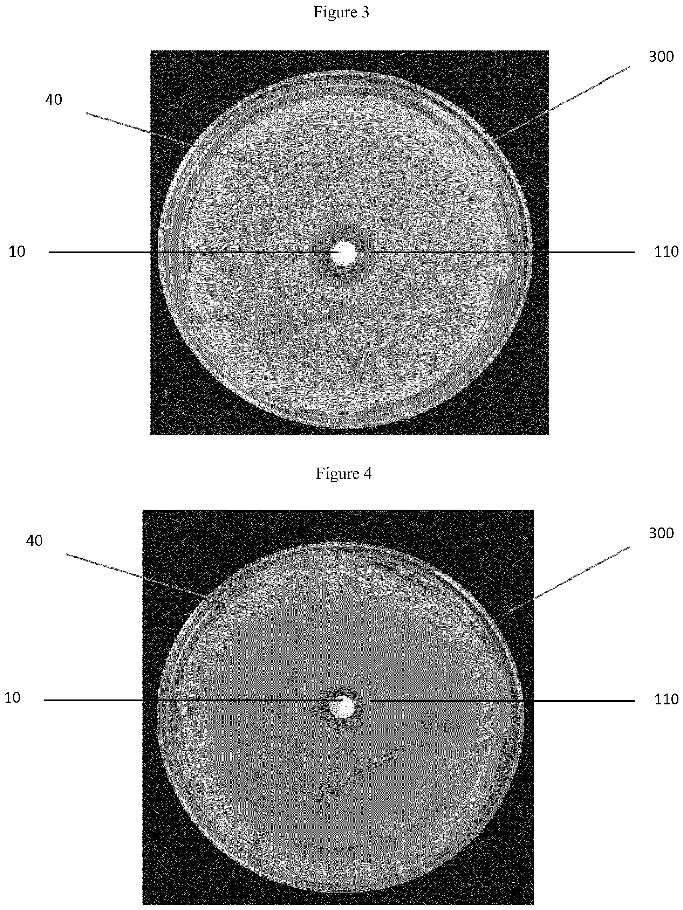

Figure 3 shows the antimicrobial properties of a bead 10 of the clinical material of the present invention showing a ZOI 110 of *Staphylococcus aureus* 40 with calcium sulfate combined with sodium diacetate and acetic acid (pH 4.83) incubated on TSA plate 300 at 33 ±2 °C for 24 hrs.
Figure 4 shows the antimicrobial properties of a bead 10 of the clinical material of the present invention showing a ZOI 110 of *Staphylococcus aureus* 40 with calcium sulfate combined with lactic acid solution and L-lactic acid sodium salt (pH 4.82) incubated on TSA plate 300 at 33 ±2 °C for 24 hrs.

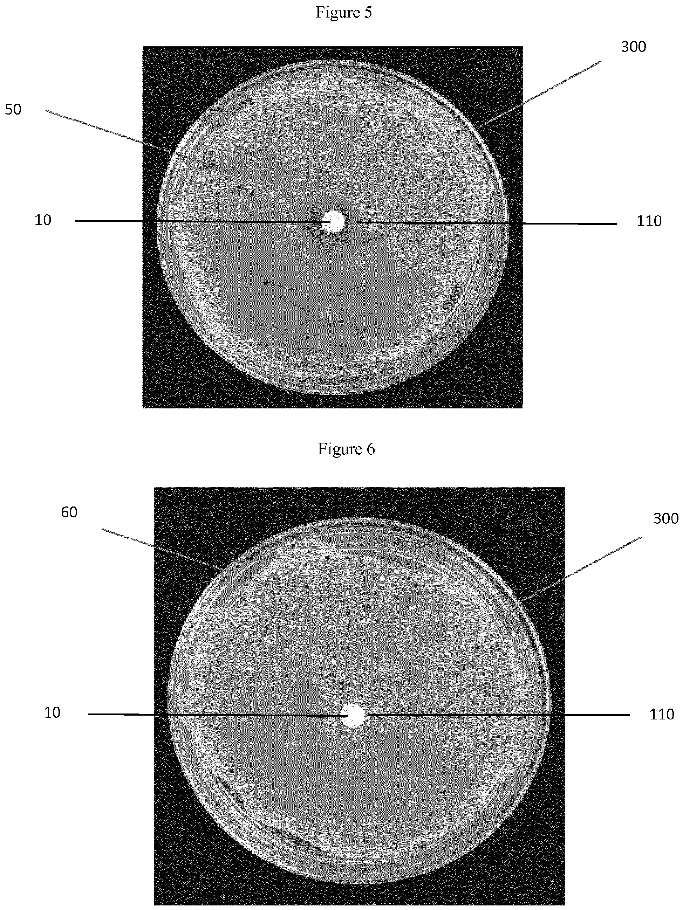

Figure 5 shows the antimicrobial properties of a bead 10 of the clinical material of the present invention showing a ZOI 110 of methicillin-resistant *Staphylococcus aureus* (MRSA) 50 with calcium sulfate combined with sodium diacetate and acetic acid (pH 4.83) incubated on TSA plate 300 at 33 ±2 °C for 24 hrs.
Figure 6 shows the antimicrobial properties of a bead 10 of the clinical material of the present invention showing a ZOI 110 of methicillin-resistant *Staphylococcus aureus* (MRSA) 60 with calcium sulfate combined with lactic acid solution and l-lactic acid sodium salt (pH 4.82) incubated on TSA plate 300 at 33 ±2 °C for 24 hrs, showing the antimicrobial properties of the clinical material.

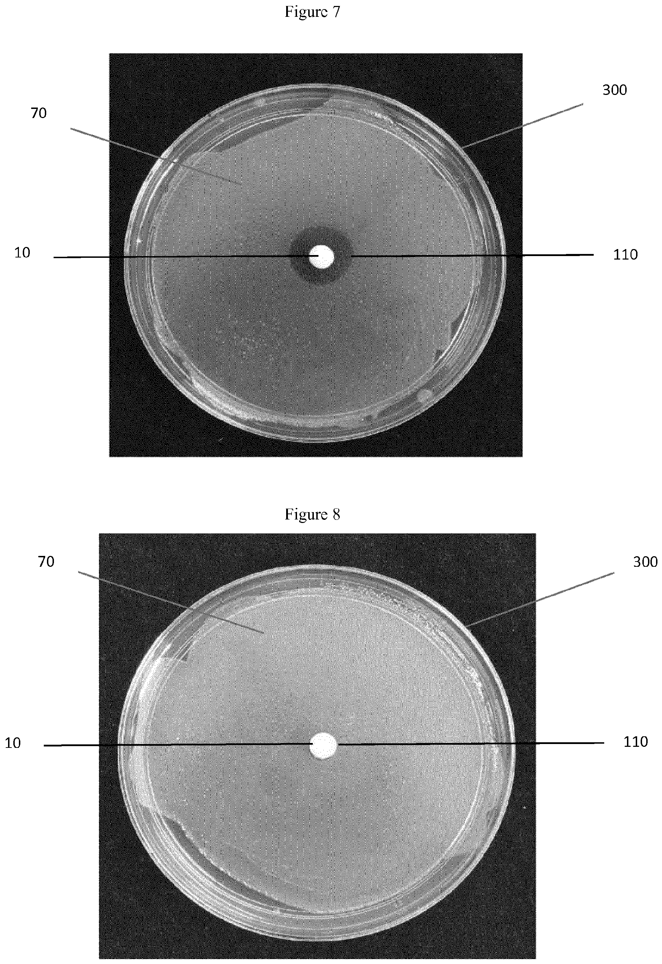

Figure 7 shows the antimicrobial properties of a bead 10 of the clinical material of the present invention showing a ZOI 110 of *Pseudomonas aeruginosa* 70 with calcium sulfate combined with sodium diacetate and acetic acid (pH 4.83) incubated on TSA plate 300 at 33 ±2 °C for 24 hrs.
Figure 8 shows the antimicrobial properties of a bead 10 of the clinical material of the present invention showing a ZOI 100 of *Pseudomonas aeruginosa* 70 with calcium sulfate combined with lactic acid solution and L-lactic acid sodium salt (pH 4.82) incubated on TSA plate 300 at 33 ±2 °C for 24 hrs.

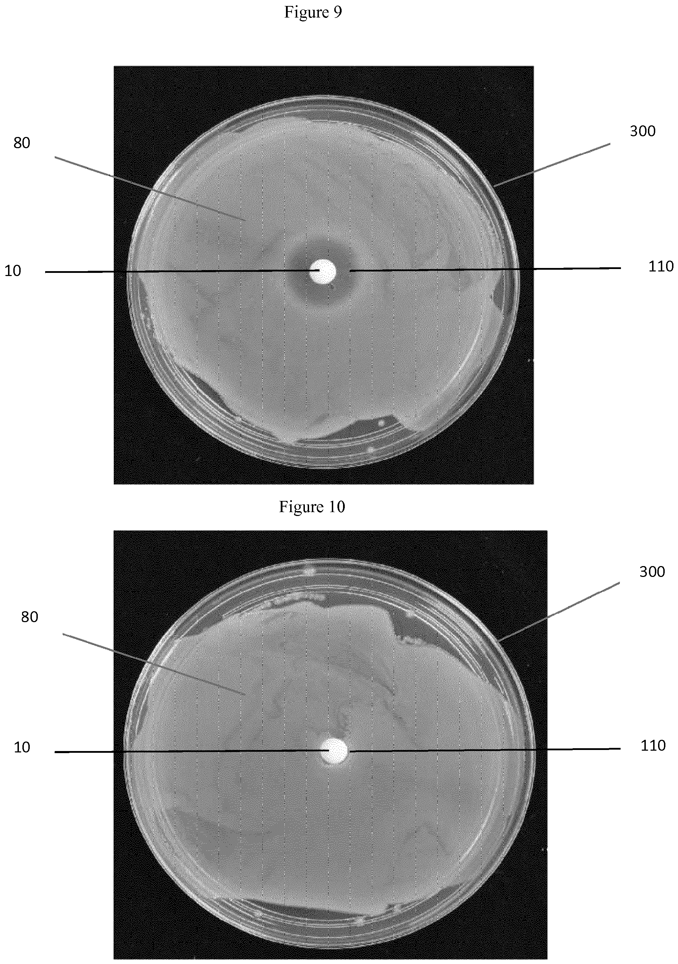

Figure 9 shows the antimicrobial properties of a bead 10 of the clinical material of the present invention showing a ZOI 110 of *Acinetobacter baumannii* 80 with calcium sulfate combined with sodium diacetate and acetic acid (pH 4.83) incubated on TSA plate 300 at 33 ±2 °C for 24 hrs.
Figure 10 shows the antimicrobial properties of a bead 10 of the clinical material of the present invention showing a ZOI 110 of *Acinetobacter baumannii* 80 with calcium sulfate combined with lactic acid solution and L-lactic acid sodium salt (pH 4.82) incubated on TSA plate 300 at 33 ±2 °C for 24 hrs.

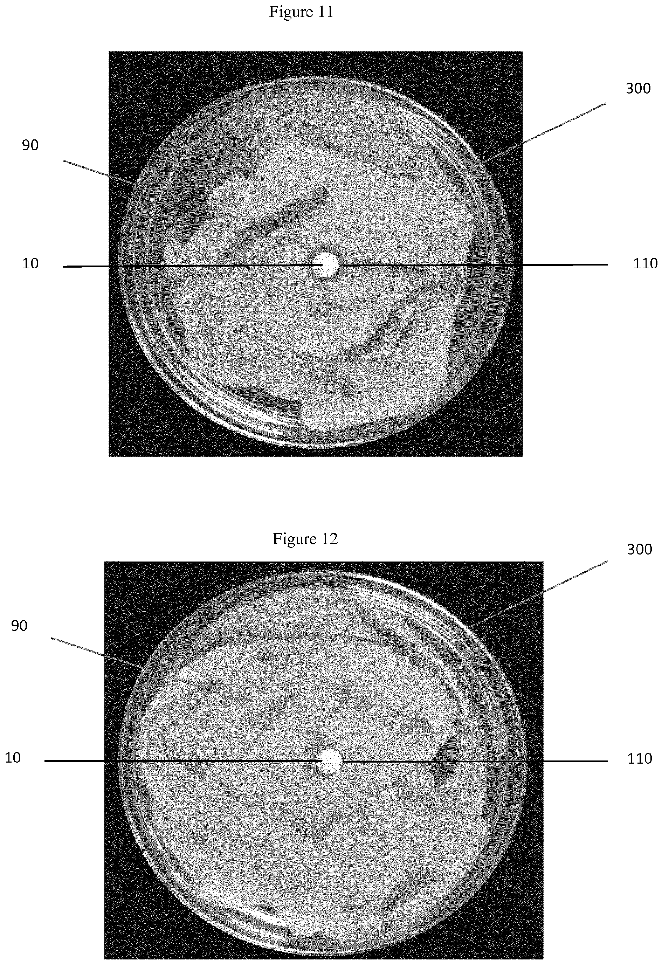

Figure 11 shows the antimicrobial properties of a bead 10 of the clinical material of the present invention showing a ZOI 110 of *Candida albicans* 90 with calcium sulfate combined with sodium diacetate and acetic acid (pH 4.83) incubated on Sabourauds Dextrose agar plate 300 at 22 ±2 °C for 48 hrs.
Figure 12 shows the antimicrobial properties of a bead 10 of the clinical material of the present invention showing a ZOI 100 of *Candida albicans* 90 with calcium sulfate combined with lactic acid solution and L-lactic acid sodium salt (pH 4.82) incubated on Sabourauds Dextrose agar plate 300 at 22 ±2 °C for 48 hrs.

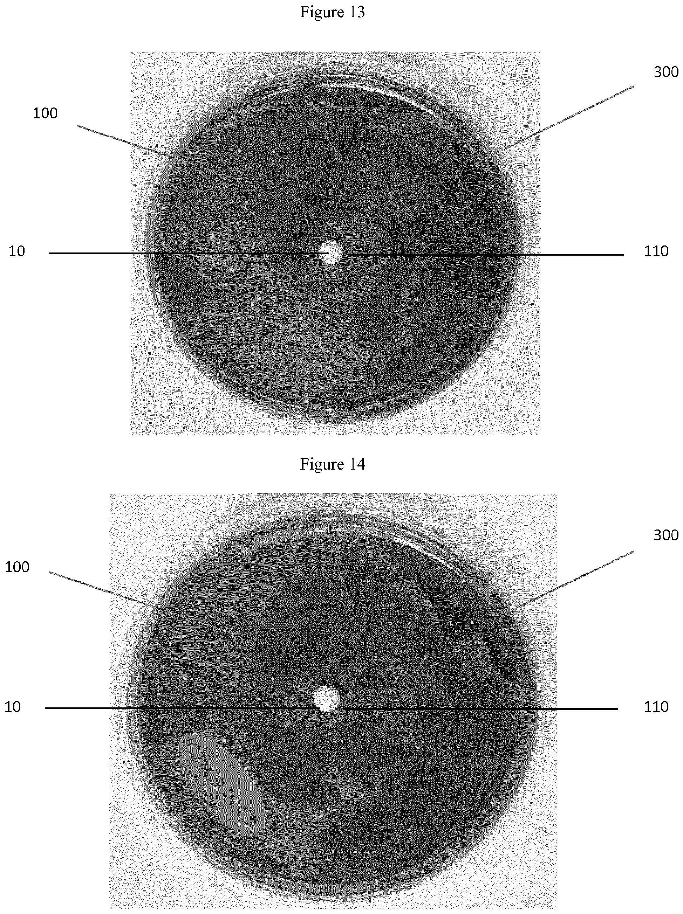

Figure 13 shows the antimicrobial properties of a bead 10 of the clinical material of the present invention showing a ZOI 110 of *Propionibacterium acnes* 100 with calcium sulfate combined with sodium diacetate and acetic acid (pH 4.83) incubated on Blood agar plate 300 at 33 ±2 °C in an anaerobic atmosphere for 5 days.
Figure 14 shows the antimicrobial properties of a bead 10 of the clinical material of the present invention showing a ZOI 110 of *Propionibacterium acnes* 100 with calcium sulfate combined with lactic acid solution and L-lactic acid sodium salt (pH 4.82) incubated on Blood agar plate 300 at 33 ±2 °C in an anaerobic atmosphere for 5 days.

In many of the Figures, a Zone of Inhibition around the bead in the centre of the dish can be observed, where the clinical material of the invention has demonstrated an antimicrobial effect and prevented the growth of the microbes in the area surrounding it.

Calcium sulfate combined with sodium diacetate and acetic acid demonstrated an antimicrobial effect against *Pseudomonas aeruginosa* and *Staphylococcus aureus*, *Staphylococcus epidermidis*, *Propionibacterium acnes* and the fungus *Candida albicans.* It reduced the growth of *Acinetobacter baumannii* and MRSA.

Calcium sulfate combined with lactic acid solution and L-lactic acid sodium salt demonstrated an antimicrobial effect against *Staphylococcus aureus*, *Staphylococcus epidermidis* and reduced the growth of *Propionibacterium acnes.* This combination has a limited effect against MRSA, *Pseudomonas aeruginosa*, *Acinetobacter baumannii* and the Fungus *Candida albicans.*

### EXAMPLES

The calcium sulfate alpha-hemihydrate powder used in the following examples was prepared from high purity, synthetic, recrystallized calcium sulfate dihydrate according to the methods described in US Patent 6,780,391 which is wholly incorporated herein by reference.

### Example 1 Control

A material was prepared according to the following recipe:
19 gm of high purity calcium sulfate alpha-hemihydrate powder were mixed with 6 ml of deionised water for about 30 seconds to form a smooth paste. The mixture was pasted in to 6 mm diameter hemispherical cavities in a flexible rubber mould where it set hard in about 10 minutes. One gram of set beads when crushed into a paste with 50ml of deionised water had a pH of 7.4. The set beads were removed from the mould simply by flexing and the beads were used for ZOI testing against a range of bacterial and fungal pathogens. No clear zones were formed with any of the pathogens challenged indicating that the calcium sulfate alone is not antimicrobial.

### Example 2

A clinical material was prepared according to the following recipe:
A homogeneous mixture containing 19 gm of high purity calcium sulfate alpha-hemihydrate powder was mixed with 6 ml of 4% acetic acid solution for about 30 seconds to form a smooth paste. The mixture was pasted in to 6 mm diameter hemispherical cavities in a flexible rubber mould where it set hard in about 10 minutes. One gram of set beads when crushed into a paste with 50ml of deionised water had a pH of 4.79.

These beads may be implanted in to hard or soft tissue sites where they provide a number of significant clinical benefits. They help to form bone in a bone site while fully resorbing in soft tissue sites. They may be applied topically to wounds where they inhibit bacterial colonisation and help prevent microbial growth both on the beads themselves and in adjacent tissues. When used topically they may help wound healing.

### Example 3

A clinical material was prepared according to the following recipe:
A homogeneous mixture containing 18 gm of high purity calcium sulfate alpha-hemihydrate powder, 0.38 gm of gypsum (calcium sulfate dihydrate) powder and 0.30 gm of sodium diacetate powder were mixed with 6 ml of 2% acetic acid solution for 30 seconds to form a smooth paste. The mixture was pasted in to 6 mm diameter hemispherical cavities in a flexible rubber mould where it set hard in about 12 minutes. One gram of set beads when crushed into a paste with 50ml of deionised water had a pH of 4.83. The set beads were removed from the mould simply by flexing and the beads were used for ZOI testing against a range of bacterial and fungal pathogens - see Tables 1-7. An antimicrobial effect was demonstrated against *Pseudomonas aeruginosa* and *Staphylococcus aureus,* Gram negative and Gram positive species.

### Example 4

A clinical material was prepared according to the following recipe:
A homogeneous mixture containing 19 gm of high purity calcium sulfate alpha-hemihydrate powder and 0.361gm of L-lactic acid sodium salt powder were mixed for 40 seconds with 6ml of 2% lactic acid solution to form a smooth paste. The mixture was pasted in to 6 mm diameter hemispherical cavities in a flexible rubber mould where it set hard in about 6 minutes. One gram of set beads when crushed into a paste with 50ml of deionised water had a pH of 4.82. The set beads were removed from the mould simply by flexing and the beads were used for ZOI testing against a range of bacterial and fungal pathogens - see Tables 1-7. An antimicrobial effect was demonstrated against *Staphylococcus aureus,* a Gram positive species ≥ the EUCAST breakpoint. Against *Staphylococcus epidermidis,* it produced an antimicrobial response but below the EUCAST breakpoints. This example also reduced the growth of *Propionibacterium acnes and h*ad a limited effect against MRSA, *Pseudomonas aeruginosa, Acinetobacter baumannii* and the fungus *Candida albicans.*

**ZOI Results against Staphylococcus epidermidis**

| Table 1 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | 13-14 | 13-14 | 12-13 |
| Calcium sulfate combined with lactic acid solution and L-lactic acid sodium salt (pH 4.82) | 8-9 | 11 | 13-14 |

**ZOI Results against Staphylococcus aureus**

| Table 2 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | 15-16 | 15-16 | 15-16 |
| Calcium sulfate combined with lactic acid solution and L-lactic acid sodium salt (pH 4.82) | 17-19 | 15-16 | 9 |

**ZOI Results against MRSA**

| Table 3 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | -ve ‡ | -ve ‡ | -ve ‡ |
| Calcium sulfate combined with lactic acid solution and L-lactic acid sodium salt (pH 4.82) | -ve | -ve | -ve |

| | | | |
|---|---|---|---|
| NOTE -ve - No Growth observed under test sample. ‡ - Zone of reduced growth measured, total size 8-10mm | | | |

**ZOI Results against Pseudomonas aeruginosa**

| Table 4 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | 15-16 * | 15-16 * | 15* |
| Calcium sulfate combined with lactic acid solution and L-lactic acid sodium salt (pH 4.82) | -ve | -ve | -ve |

| | | | |
|---|---|---|---|
| NOTE -ve - No Growth observed under test sample. * Isolated colonies observed in clear zone. | | | |

**ZOI Results against Acinetobacter baumannii**

| Table 5 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | -ve ‡ | -ve ‡ | -ve ‡ |
| Calcium sulfate combined with lactic acid solution and | -ve | -ve | -ve |
| L-lactic acid sodium salt (pH 4.82) | | | |

| | | | |
|---|---|---|---|
| NOTE -ve - No Growth observed under test sample ‡ - Zone of reduced growth measured, total size 13-15mm | | | |

**ZOI Results against Candida albicans**

| Table 6 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | 7-8 | 6-7 | 6-7 |
| Calcium sulfate combined with lactic acid solution and L-lactic acid sodium salt (pH 4.82) | -ve | -ve | -ve |

| | | | |
|---|---|---|---|
| NOTE -ve - No Growth observed under test sample | | | |

**ZOI Results against Propionibacterium acnes**

| Table 7 | Total zone diameter measured including sample (mm) | | |
|---|---|---|---|
| | Sample 1 | Sample 2 | Sample 3 |
| Calcium sulfate combined with sodium diacetate and acetic acid (pH 4.83) | 9-10 | 8-9 | 8-9 |
| Calcium sulfate combined with lactic acid solution and L-lactic acid sodium salt (pH 4.82) | -ve ‡ | -ve ‡ | -ve ‡ |

| | | | |
|---|---|---|---|
| NOTE -ve - No Growth observed under test sample. ‡ - Zone of reduced growth measured, total size 9-12mm | | | |

A number of embodiments of the present invention have been described. However, it is to be understood that various modifications may be made without departing from the scope of the invention. The organic acids may be different to those disclosed. The clinical material may comprise calcium carbonate or other materials often used as absorbable fillers in hard or soft tissue sites. The clinical material may contain a combination of different carboxylic acids and carboxylic acid salts. The carboxylic acid(s) may be mono-basic, di-basic- or tri-basic. The ammonium cation NH₃⁺ may substitute for the sodium or potassium of the carboxylic acid salts. The pH of the wet mixture may be different to that shown in the Examples. The hydrating solution may contain some or all of the alkali or alkaline earth carboxylic acid salt. The calcium sulfate hemihydrate may be the alpha or beta form or a mixture of both. The calcium sulfate may be, in whole or in part, anhydrous calcium sulfate. The clinical material may be applied as a coating to the surface of a medical implant.

### References

1. HAI Prevalence Survey 2014. https://www.cdc.gov/hai/surveillance/
2. Scott, R. Douglous, II. "The Direct Medical Costs of Healthcare-Associated Infections in U.S. Hospitals and the Benefits of Prevention." The Direct Medical Costs of Healthcare-Associated Infections in U.S. Hospitals and the Benefits of Prevention, March 2009. March 2009. https://www.cdc.gov/HAI/pdfs/hai/Scott_CostPaper.pdf.
3. Frykberg RG, Armstrong DG, Giurini J. et al. Diabetic foot disorders: a clinical practice guideline. American College of Foot and Ankle Surgeons. J Foot Ankle Surg. 2000.
4. David J Margolis, MD, PhD, D Scot Malay, DPM, MSCE, Ole J Hoffstad, MA, Charles E Leonard, PharmD, Thomas MaCurdy, PhD, Yang Tan, BA, Teresa Molina, BA, Karla Lopez de Nava, PhD, and Karen L Siegel, PT, MA. (2011) Economic Burden of Diabetic Foot Ulcers and Amputations. https://www.ncbi.nlm.nih.gov/books/NBK65152/
5. Leone S, Pascale R, Vitale M, Esposito S. [Epidemiology of diabetic foot] Infez Med. 2012;20 Suppl 1:8-13. Leone S, Pascale R, Vitale M, Esposito S. [Epidemiology of diabetic foot] Infez Med. 2012;20 Suppl 1:8-13.
6. Ventola CL. The Antibiotic Resistance Crisis: Part 1: Causes and Threats. Pharmacy and Therapeutics. 2015;40(4):277-283.
7. Drago L, Vassena C, Fenu S, De Vecchi E, Signori V, De Francesco R, Romanò CL. In vitro antibiofilm activity of bioactive glass S53P4. Future Microbiol. 2014;9(5):593-601. doi: 10.2217/fmb.14.20. PubMed PMID: 24957087.
8. MACA, J. V., MILLER, R. K. and ACUFF, G. R. (1997), Microbiological, Sensory and Chemical Characteristics of Vacuum-Packaged Ground Beef Patties Treated with Salts of Organic Acids. Journal of Food Science, 62: 591-596.
9. Sallam, K. I., & Samejima, K. (2004). Microbiological and chemical quality of ground beef treated with sodium lactate and sodium chloride during refrigerated storage. Lebensmittel-Wissenschaft + [i.e. Und] Technologie. Food Science + Technology. Science + Technologie Alimentaire, 37(8), 865-871.
10. Williams SK, Phillips K. Sodium lactate affects sensory and objective characteristics of tray-packed broiler chicken breast meat. Poult Sci. 1998 May;77(5):765-9. PubMed PMID: 9603367.
11. G. Boskou & J. Debevere, Shelf-life extension of cod fillets with an acetate buffer spray prior to packaging under modified atmospheres. Food Additives and Contaminants. Volume 17, 2000 - Issue 1 Pages 17-25.
12. ZHUANG, R.-Y., HUANG, Y.-W. and BEUCHAT, L. R. (1996), Quality Changes During Refrigerated Storage of Packaged Shrimp and Catfish Fillets Treated with Sodium Acetate, Sodium Lactate or Propyl Gallate. Journal of Food Science, 61: 241-244.
13. Sallam KI. Chemical, sensory and shelf life evaluation of sliced salmon treated with salts of organic acids. Food chemistry. 2007;101(2):592-600. doi:10.1016/j.foodchem.2006.02.019.
14. Lee YL, Cesario T, Owens J, Shanbrom E, Thrupp LD. Antibacterial activity of citrate and acetate. Nutrition. 2002 Jul-Aug;18(7-8):665-6. PubMed PMID:12093451.
15. Qvist S, Sehested K, Zeuthen P. Growth suppression of Listeria monocytogenes in a meat product. Int J Food Microbiol. 1994 Dec;24(1-2):283-93. PubMed PMID:7703021.
16. McWilliam Leitch EC, Stewart CS. Susceptibility of Escherichia coli 0157 and non-0157 isolates to lactate. Lett Appl Microbiol. 2002;35(3):176-80. PubMedPMID: 12180936.
17. Anders R.J., Cerveny, J.G., Milkowski, A.L., Method for delaying Clostridium botulinum growth in fish and poultry US Patent 4,798,729.
18. Wannee Tangkham , Jay Comeaux , Clarence E. Ferguson and Frederick M. LeMieux., Effect of sodium lactate and sodium acetate on shelf-life of raw chicken breasts. African Journal of Food Science Vol. 6(13), pp. 375-380, 15 July, 2012
19. Nuñez de Gonzalez MT1, Keeton JT, Acuff GR, Ringer LJ, Lucia LM. Effectiveness of Acidic Calcium Sulfate with Propionic and Lactic Acid and Lactates as Postprocessing Dipping Solutions To Control Listeria monocytogenes on Frankfurters with or without Potassium Lactate and Stored Vacuum Packaged at 4.5°C. Journal of Food Protection 2004, 67: 915-921
20. Basavraj S. Nagoba, Namdev M. Suryawanshi, Bharat Wadher, Sohan Selkar., Acidic Environment and Wound Healing: A Review., WOUNDS. 2015;27(1):5-11.
21. The European Committee on Antimicrobial Susceptibility Testing. Breakpoint tables for interpretation of MICs and zone diameters. Version 7.0, 2017. http://www.eucast.org.
22. Jan Hudzicki. 2009. Kirby-bauer disk diffusion susceptibility test protocol.

## Claims

1. A clinical material suitable for use within or on a body of a patient, the material comprising a resorbable carrier material having antimicrobial properties, the carrier material comprising one of acidified calcium sulfate, a calcium phosphate, collagen, cancellous and cortical allograft bone, cancellous and cortical autograft bone, a resorbable polymer, or a composite material comprising any two or more thereof.

2. A clinical material according to claim 1, further comprising a carboxylic salt of an alkali or alkaline earth metal.

3. A clinical material according to claim 2, which further comprises a carboxylic acid.

4. A clinical material according to claim 3, where the carboxylic acid has a molecular mass in the range of about 40 to about 200 daltons.

5. A clinical material according to any preceding claim, wherein the carboxylic acid is selected from acetic acid, propionic acid, valeric acid, butyric acid, benzoic acid, malic acid, ascorbic acid, glycolic acid, lactic acid, formic acid, caproic acid, enanthic acid, caprylic acid, pelargonic acid, capric acid, undecylic acid and lauric acid, sorbic acid or a combination of any two or more thereof.

6. A clinical material according to claim 5, where the carboxylic acid is acetic acid and/or lactic acid.

7. A clinical material according to any preceding claim, wherein the resorbable carrier material comprises acidified calcium sulfate and/or a calcium phosphate.

8. A clinical material according to any preceding claim, wherein the calcium sulfate is selected from the group comprising of calcium sulfate hemihydrate, calcium sulfate dihydrate or anhydrous calcium sulfate.

9. A clinical material according to claim 8 where the calcium sulfate is about 40 wt% to about 100 wt% of the clinical material, optionally, about 90 wt% to about 98 wt% of the clinical material.

10. A clinical material according to any preceding claim where the clinical material has a pH in the range of 3 to 7, optionally in the range of 4 to 5.

11. A clinical material according to claim 2 where the carboxylic salt is an alkali or alkaline earth metal salt of a carboxylic acid having a molecular mass of about 40 to about 200 daltons, and is selected from the group comprising of sodium, potassium, magnesium or calcium cations.

12. A clinical material according to claim 13 where the carboxylic salt is an acetate, ascorbate, benzoate, butyrate, citrate, formate, glycolate, lactate, malate, oxalate, propionate, salicylate, sorbate, tartrate or valerate salt, alone or in combination.

13. A clinical material according to claim 14 where the salt is an acetate salt on its own or in combination with a lactate salt.

14. A clinical material according to any preceding claim which is absorbable within or on a patient's body over a period of about 2 to about 12 weeks; optionally between about 4 to about 12 weeks when implanted in hard tissue, or within about 2 to about 4 weeks when placed in a soft tissue site.

15. A kit of parts for the production of a clinical material having antimicrobial properties for the treatment and prevention of infection within or on a patient's body; the kit of parts comprising a resorbable carrier material having antimicrobial properties, the carrier material comprising one of acidified calcium sulfate, a calcium phosphate, collagen, cancellous and cortical allograft bone, cancellous and cortical autograft bone, a resorbable polymer, or a composite material comprising any two or more thereof; an alkali or alkaline earth metal salt of a low molecular mass carboxylic acid; and a carboxylic acid hydrating solution.
